# EUROPEAN PATENT APPLICATION

(11) **EP 3 369 359 A1**
(43) Date of publication of application: **05.09.2018**
(21) Application number: 15907225.5
(22) Date of filing: 27.10.2015
(51) Int. Cl.: A61B 1/04

(54) **IMAGING DEVICE AND ENDOSCOPE**

(71) Applicant: Olympus Corporation, Hachioji-shi, Tokyo 192-8507 (JP)
(72) Inventor: YOSHIDA, Kazuhiro, Tokyo 192-8507 (JP)
(74) Representative: Winter, Brandl, Fürniss, Hübner, Röss, Kaiser, Polte - Partnerschaft mbB
(86) International application number: PCT/JP2015/080275
(87) International publication number: WO 2017/072861

(57) **Abstract**

Provided are an imaging unit and an endoscope with reduced diameters and high moisture resistance. An imaging apparatus 100 includes a lens unit 40 in which a plurality of optical elements 40a-1 to 40a-3 including one or more wafer-level lenses and a spacer 40b are laminated, an imaging element 51 configured to receive light incident from the lens unit 40, perform photoelectric conversion on the light, and generate an image signal, and a moisture-proof member 54 that is bent and arranged so as to come in contact with at least a part of side surfaces of the lens unit 40 to prevent moisture from entering a gap between the optical element 40a and the spacer 40b and the like.

## Description

### Field

The present invention relates to an imaging apparatus that is inserted into a subject, captures an image inside the subject, and generates image data inside the subject, and an endoscope including the imaging apparatus at a distal end portion thereof.

### Background

Conventionally, an endoscope acquires an in-vivo image of a subject, such as a patient, by inserting a long and thin flexible insertion portion, which is provided with an imaging apparatus at a distal end thereof, into the subject. The imaging apparatus used in the endoscope as described above includes a refractive optical system that includes an optical lens group and a prism, an imaging element that performs photoelectric conversion on light incident from the refractive optical system and generates an image signal, a lens holding frame and a prism holding frame for holding the optical lens group and the prism, respectively, and a light shielding member that is mounted so as to cover the refractive optical system (see, for example, Patent Literature 1).

### Citation List

### Patent Literature

Patent Literature 1: Japanese Patent No. 5080695 Summary

### Technical Problem

In Patent Literature 1 described above, the optical lens group and the prism are respectively held by the lens holding frame and the prism holding frame, and therefore, a size of the imaging apparatus in a radial direction is increased due to the thicknesses of the lens holding frame and the prism holding frame. If the imaging apparatus as described above is used in the endoscope, a burden on a patient at the time of insertion may increase. Therefore, there is a need for an imaging apparatus whose diameter can be reduced in order to reduce a burden on a patient.

To solve the problem as described above, an imaging module for mobile phones, which does not use a lens holding frame and which has recently been developed, may be applied to endoscopes. The imaging module for mobile phones does not use a lens frame by laminating wafer-level lenses whose sizes can be reduced. However, because an endoscope is used inside a subject, it is necessary to improve moisture resistance of the imaging apparatus in order to prevent fog or the like on the wafer-level lenses whose side surfaces are exposed.

The present invention has been conceived in view of the foregoing situations, and an object is to provide an imaging apparatus and an endoscope with reduced diameters and improved moisture-proof performance.

### Solution to Problem

To solve the above-described problem and achieve the object, an imaging apparatus according to the present invention includes: a lens unit in which a plurality of optical elements including one or more wafer-level lenses and a spacer are laminated; an imaging element configured to receive light incident from the lens unit, perform photoelectric conversion on the light, and generate an image signal; and a moisture-proof member that is bent and arranged so as to come in contact with at least a part of side surfaces of the lens unit to prevent moisture from entering a gap between the optical element and the spacer and/or a gap between the optical elements.

Moreover, in the above-described imaging apparatus according to the present invention, the moisture-proof member is a flexible printed circuit board and bonded to the side surfaces of the lens unit by an adhesive agent.

Moreover, in the above-described imaging apparatus according to the present invention, the moisture-proof member is a flexible connector including a base material with moisture-proof property and an adhesive layer provided on a surface of the base material.

Moreover, in the above-described imaging apparatus according to the present invention, the moisture-proof member is a flexible connector including a base material with moisture-proof property, and an adhesive layer and a wiring layer that are provided on a surface of the base material.

Moreover, in the above-described imaging apparatus according to the present invention, the imaging element is arranged such that a principal surface, on which the light receiving unit is provided, is arranged perpendicular to an optical axis of the lens unit.

Moreover, in the above-described imaging apparatus according to the present invention, the moisture-proof member is a flexible connector including a base material with moisture-proof property, an adhesive layer provided on a surface of the base material, and a moisture-resistant ring for sealing a boundary between the optical element and the spacer and/or a boundary between the optical elements.

Moreover, in the above-described imaging apparatus according to the present invention, the moisture-proof member is a flexible connector including a base material with moisture-proof property, an adhesive layer provided on a surface of the base material, and a heater wiring that heats the lens unit.

Moreover, the above-described imaging apparatus according to the present invention further includes: a prism provided on the light receiving unit and configured to reflect light incident from the lens unit toward the light receiving unit, wherein the imaging element is arranged such that a principal surface on which the light receiving unit is provided is arranged parallel to an optical axis of the lens unit, and the lens unit is arranged on the imaging element, the moisture-proof member is a flexible connector that includes a base material with moisture-proof property, an adhesive layer provided on a surface of the base material, and a wiring layer, and is bent and arranged such that the adhesive layer and the wiring layer come in contact with the lens unit, and the wiring layer is connected to an electrode pad of the imaging element via a through via provided in the moisture-proof member.

Moreover, in the above-described imaging apparatus according to the present invention, the moisture-proof member includes a light shielding layer on a surface opposite to a surface that comes in contact with the lens unit.

Moreover, the above-described imaging apparatus according to the present invention further includes: a flexible printed circuit board on which a signal cable and an electronic component are mounted and which is connected to an electrode pad of the imaging element, wherein the moisture-proof member is integrated with the flexible printed circuit board.

Moreover, an endoscope according to the present invention includes: any one of the above-described imaging apparatuses; and an insertion portion that includes a tubular distal end portion made of a rigid material and that is insertable into a subject, wherein the insertion portion is provided with the imaging apparatus in an internal space of the distal end portion.

### Advantageous Effects of Invention

According to the present invention, it is possible to obtain an imaging unit and an endoscope with high moisture-proof performance and reduced diameters. Brief Description of Drawings

FIG. 1 is a diagram schematically illustrating an overall configuration of an endoscopic system according to a first embodiment of the present invention.
FIG. 2 is a partial cross-sectional view of a distal end portion of an endoscope according to the first embodiment of the present invention.
FIG. 3 is a cross-sectional view of an imaging apparatus according to the first embodiment of the present invention.
FIG. 4A is a perspective view for explaining arrangement of a moisture-proof member of the imaging apparatus in the first embodiment of the present invention (before arrangement).
FIG. 4B is a perspective view for explaining arrangement of the moisture-proof member of the imaging apparatus in the first embodiment of the present invention (after arrangement).
FIG. 5A is a perspective view for explaining arrangement of a moisture-proof member of an imaging apparatus according to a first modification of the first embodiment of the present invention (before arrangement).
FIG. 5B is a perspective view for explaining arrangement of the moisture-proof member of the imaging apparatus according to the first modification of the first embodiment of the present invention (after arrangement).
FIG. 5C is an enlarged view of a connection portion of an imaging element and a moisture-proof member 54A according to the first modification of the first embodiment of the present invention.
FIG. 6A is a cross-sectional view of an imaging apparatus according to a second embodiment of the present invention.
FIG. 6B is a perspective view for explaining arrangement of a moisture-proof member of the imaging apparatus in the second embodiment of the present invention (before arrangement).
FIG. 6C is a perspective view for explaining arrangement of the moisture-proof member of the imaging apparatus in the second embodiment of the present invention (after arrangement).
FIG. 7 is a perspective view for explaining arrangement of a moisture-proof member of an imaging apparatus in a third embodiment of the present invention (before arrangement).

### Description of Embodiments

Reference will be made below to an endoscope including an imaging apparatus as modes for carrying out the present invention (hereinafter, referred to as "embodiments"). The present invention is not limited by the embodiments below. The drawings referred to in the description below schematically illustrate shapes, sizes, and positional relationships merely to make contents of the present invention understandable. That is, the present invention is not limited only to the shapes, the sizes, and the positional relationships illustrated in the individual drawings. Different drawings may include parts with dimensions or ratios being different from one another.

### (First Embodiment)

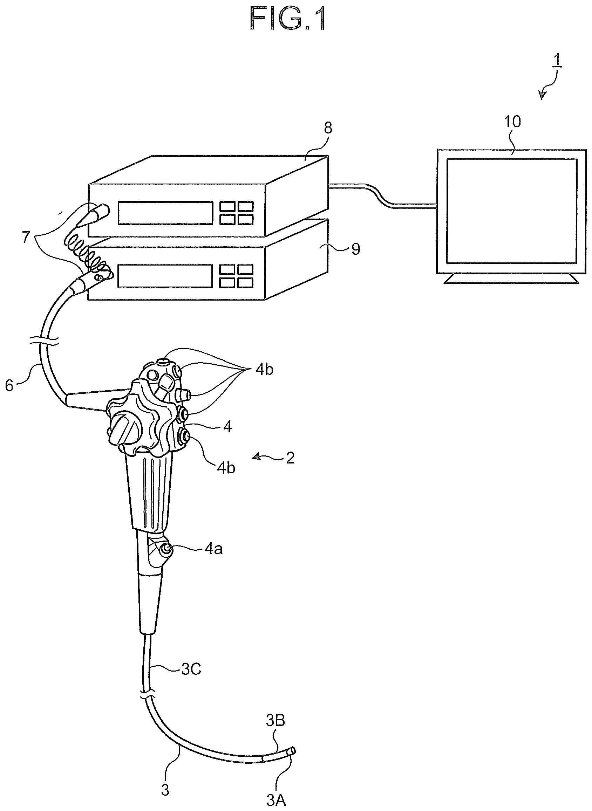

FIG. 1 is a diagram schematically illustrating an overall configuration of an endoscopic system according to a first embodiment of the present invention. An endoscopic system 1 illustrated in FIG. 1 includes an endoscope 2, a universal cord 6 (transmission cable), a connector 7, a processor 8 (control device), a light source device 9, and a display device 10.

The endoscope 2 captures an in-vivo image of a subject and outputs an image signal (image data) to the processor 8 by inserting an insertion portion 3 into the subject. An electric cable bundle within the universal cord 6 is extended to the insertion portion 3 of the endoscope 2 and connected to an imaging apparatus provided at a distal end portion 3A of the insertion portion 3. An operating unit 4 including various buttons and knobs or the like for operating endoscope functions is connected to a proximal end side of the insertion portion 3 of the endoscope 2. A treatment tool insertion opening 4a, which is for inserting a treatment tool such as a biopsy forceps, an electric scalpel, and an inspection probe into a body cavity of the subject, is provided in the operating unit 4.

The connector 7 is provided at a proximal end of the universal cord 6 and connected to the processor 8 and the light source device 9. The connector 7 performs predetermined signal processing on the image signal output from the imaging apparatus at the distal end portion 3A connected to the universal cord 6, performs analog-to-digital (A/D) conversion on the image signal, and outputs the digital image signal to the processor 8.

The processor 8 performs predetermined image processing on the image signal output from the connector 7 and outputs the image signal to the display device 10. Further, the processor 8 controls the whole endoscopic system 1. The processor 8 is constituted by a central processing unit (CPU) or the like.

The display device 10 displays an image corresponding to the image signal output from the processor 8. The display device 10 is constituted by a display panel or the like made of liquid crystal, organic electro luminescence (EL), or the like.

The light source device 9 emits illumination light from the distal end of the insertion portion 3 of the endoscope 2 toward the subject via the connector 7 and the universal cord 6. The light source device 9 is constituted by a xenon lamp, a light emitting diode (LED) lamp, or the like.

The insertion portion 3 includes the distal end portion 3A at which the imaging apparatus is provided, a bendable portion 3B that is continuously provided at a proximal end side of the distal end portion 3A and bendable in a plurality of directions, and a flexible tube 3C that is continuously provided at a proximal end side of the bendable portion 3B. An image signal obtained by imaging by the imaging apparatus provided at the distal end portion 3A is connected to the connector 7 by the universal cord 6, which has a length of a few meters for example, via the operating unit 4. The bendable portion 3B is bent by operation of a bending operation knob 4b provided in the operating unit 4, and bendable in four directions such as upward, downward, leftward, and rightward for example along with pulling and loosening of a bending wire inserted into the insertion portion 3.

In addition, the endoscope 2 includes a light guide (not illustrated) for propagating illumination light from the light source device 9, and an illumination lens (not illustrated) that is arranged at an emission end of the illumination light delivered through the light guide. The illumination lens is provided at the distal end portion 3A of the insertion portion 3.

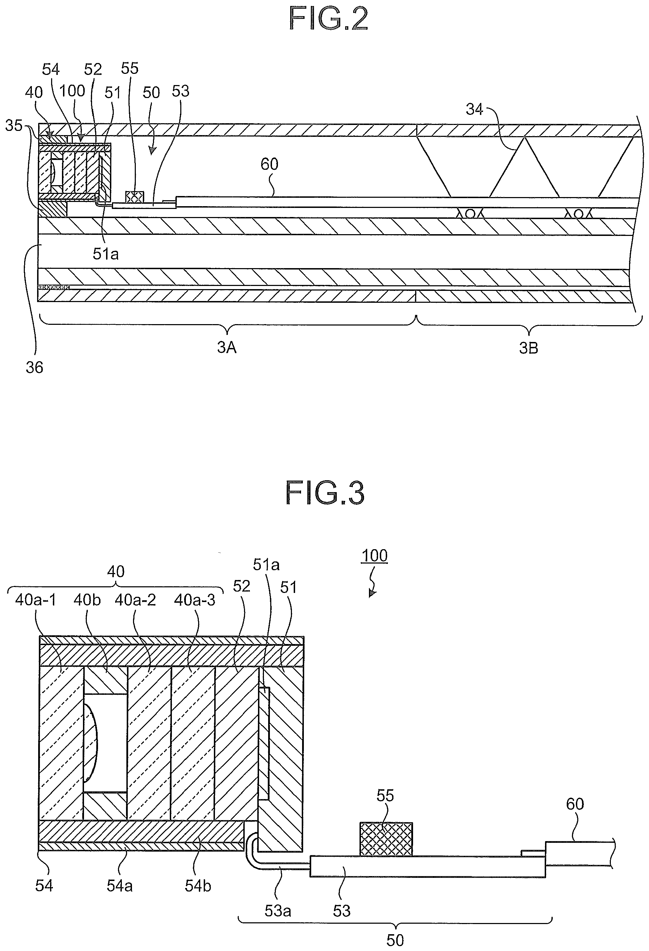

Next, a configuration of the distal end portion 3A of the endoscope 2 will be described in detail. FIG. 2 is a partial cross-sectional view of the distal end portion 3A of the endoscope 2, which is a cross section cut by a plane that is perpendicular to a substrate surface of the imaging apparatus provided at the distal end portion 3A of the endoscope 2 and that is parallel to an optical axis direction of the imaging apparatus. Further, in FIG. 2, the distal end portion 3A and a part of the bendable portion 3B of the insertion portion 3 of the endoscope 2 are illustrated.

As illustrated in FIG. 2, the bendable portion 3B is bendable in the four directions such as upward, downward, leftward, and rightward along with pulling and loosening of the bending wire inserted into a bending tube 34. An imaging apparatus 100 is provided at an upper part of the distal end portion 3A that is extended from the distal end side of the bendable portion 3B, and a treatment tool channel 36 for extending various treatment tools is provided at a lower part.

The imaging apparatus 100 includes a lens unit 40 that condenses incident light, and an imaging unit 50 provided at a proximal end side of the lens unit 40. The imaging apparatus 100 is bonded to the inside of the distal end portion 3A by an adhesive agent. The distal end portion 3A is made of a rigid member for forming an internal space in which the imaging apparatus 100 is housed. An outer peripheral portion of the proximal end of the distal end portion 3A is covered with a flexible covering pipe (not illustrated). Members on the proximal end side of the distal end portion 3A are made of a flexible member such that the bendable portion 3B can be bent.

The lens unit 40 includes a plurality of optical elements 40a-1 to 40a-3, a spacer 40b disposed between the optical elements 40a-1 and 40a-2, and a diaphragm member (not illustrated) (see FIG. 3). The optical elements 40a-1 to 40a-3 are configured with wafer level lenses, for which lenses are molded at the wafer level, filters, and the like. The spacer is made of silicon, glass, or the like, and molded by ICP etching, sandblasting, or the like. The optical elements 40a-1 to 40a-3 and the spacer 40b are laminated at the wafer level and subjected to separation by blade dicing or the like to obtain the lens unit 40. A moisture-proof member 54 constituted by a flexible connector is provided on side surfaces of the lens unit 40. The lens unit 40 is fixed to the distal end portion 3A by inserting and fixing an end portion thereof in a distal end fixing portion 35 inside the distal end portion 3A. In this case, an outer diameter of the distal end of the endoscope can be reduced by reducing diameters of the lens unit 40 and the moisture-proof member 54.

An imaging element 51 includes a light receiving unit 51a that receives light emitted by the lens unit 40 and performs photoelectric conversion to generate an electrical signal, and a circuit unit (not illustrated) including an amplifier, such as a transistor. The imaging element 51 is bonded to the lens unit 40 via a cover glass 52 that protects the light receiving unit 51a of the imaging element 51. An electrode pad (not illustrated) is provided at a lower part of the imaging element 51, and a flexible printed circuit board 53 (hereinafter, also referred to as an "FPC board"), to which signal cables 60 are connected, is connected via an inner lead 53a (see FIG. 3). An electronic component 55 or the like for driving the imaging element 51 is mounted on the FPC board 53. The imaging element 51 in the first embodiment of the present invention is a semiconductor imaging element of a charge coupled device (CCD) type or a complementary metal oxide semiconductor (CMOS) type.

A proximal end of each of the signal cables 60 extends toward the proximal end of the insertion portion 3. An electric cable bundle is inserted and arranged in the insertion portion 3 and extended to the connector 7 via the operating unit 4 and the universal cord 6 illustrated in FIG. 1. The signal cables 60 are connected to the imaging element 51 via an electrode portion of the FPC board 53 to transmit a driving signal to the imaging element 51 and transmit an electrical signal corresponding to an image captured by the imaging element 51 to the processor 8.

Light incident from one end of the lens unit 40 is collected by the optical elements 40a-1 to 40a-3 and enters the imaging element 51. The light receiving unit 51a selected from a CCD image sensor, a CMOS image sensor, and the like converts the received light to an imaging signal. The imaging signal is output to the processor 8 via the signal cables 60 connected to the FPC board 53 and the connector 7. In this specification, a light incident side of the lens unit 40, that is, a side where the optical elements 40a-1 to 40a-3 are arranged, will be referred to as a front end, and a side where the signal cables 60 are arranged will be referred to as a rear end.

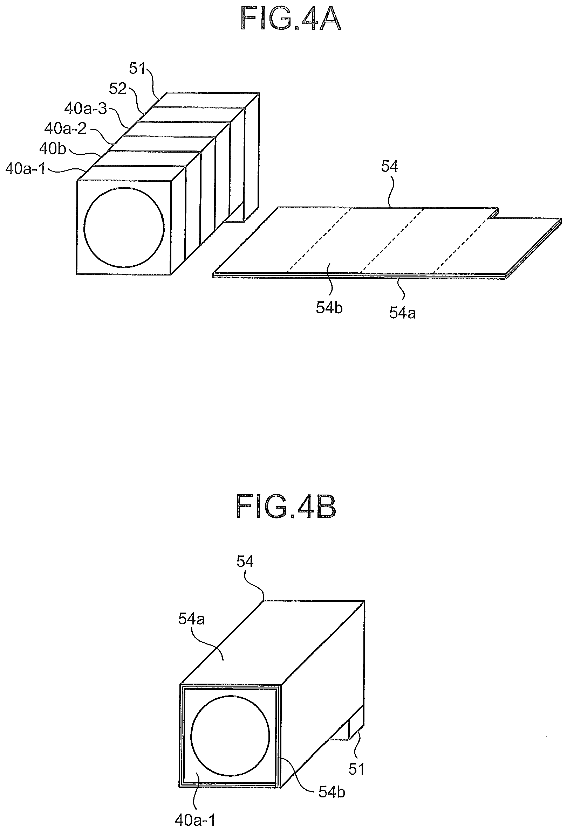

Next, the imaging apparatus 100 will be described in detail. FIG. 3 is a cross-sectional view of the imaging apparatus 100 according to the first embodiment of the present invention. FIG. 4A is a perspective view for explaining arrangement of the moisture-proof member 54 of the imaging apparatus 100 (before arrangement), and FIG. 4B is a perspective view for explaining arrangement of the moisture-proof member 54 of the imaging apparatus 100 (after arrangement). In FIG. 4A and 4B, illustrations of the FPC board 53 and the like are omitted.

In the imaging apparatus 100, as illustrated in FIG. 3, the moisture-proof member 54 is arranged so as to cover a part of outer peripheries of the lens unit 40, the cover glass 52, and the imaging element 51. The moisture-proof member 54 used in the first embodiment is a flexible connector including a base material 54a with moisture-proof property and an adhesive layer 54b provided on a surface of the base material.

The base material 54a is selected from flexible materials with moisture-proof property. For example, polyimide, liquid crystal polymer (LCP), or the like may be used. LCP is preferable because it has excellent moisture-proof property. Further, the moisture-proof member 54 is bent as indicated by dotted lines in FIG. 4A, and arranged so as to come in contact with the side surfaces of the lens unit 40 as illustrated in FIG. 4B.

As the adhesive layer 54b, a material that needs to be cured with heat or ultraviolet light or a material that does not need such curing may be used. The moisture-proof member 54 is tightly bonded to the lens unit 40 by the adhesive layer 54b, and prevents moisture from entering a gap between the optical element 40a-1 and the spacer 40b, a gap between the spacer 40b and the optical element 40a-2, and a gap between the optical element 40a-2 and the optical element 40a-3.

The moisture-proof member 54 can be bonded to the lens unit 40 by bonding the moisture-proof member 54 to the lens unit 40 through pressure bonding or by arranging and temporarily fixing the moisture-proof member 54 on the side surfaces of the lens unit 40 and then causing the adhesive layer 54b to cure with heat or ultraviolet light.

In the first embodiment, it is possible to reduce the diameter of the imaging apparatus 100 by using a wafer-level lens as the lens unit 40, and it is possible to improve the moisture-proof performance of the imaging apparatus 100 by arranging the moisture-proof member 54 on the side surfaces of the lens unit 40.

While a flexible connector is used as the moisture-proof member 54 in the first embodiment, the embodiments are not limited to this example, and it may be possible to use a moisture-resistant flexible member as a moisture-proof member. For example, the FCP board 53 may be used as the moisture-proof member 54 by bonding it to the side surfaces of the lens unit 40 by an adhesive agent.

In addition, it may be possible to provide a light shielding layer on a surface of the base material 54a opposite to the surface where the adhesive layer 54b is provided in the moisture-proof member 54. By providing the light shielding layer, it becomes possible to prevent stray light from entering the lens unit 40 and reduce influence of external light. As the light shielding layer, for example, black solder resist or metal film may be used.

### (First Modification of First Embodiment)

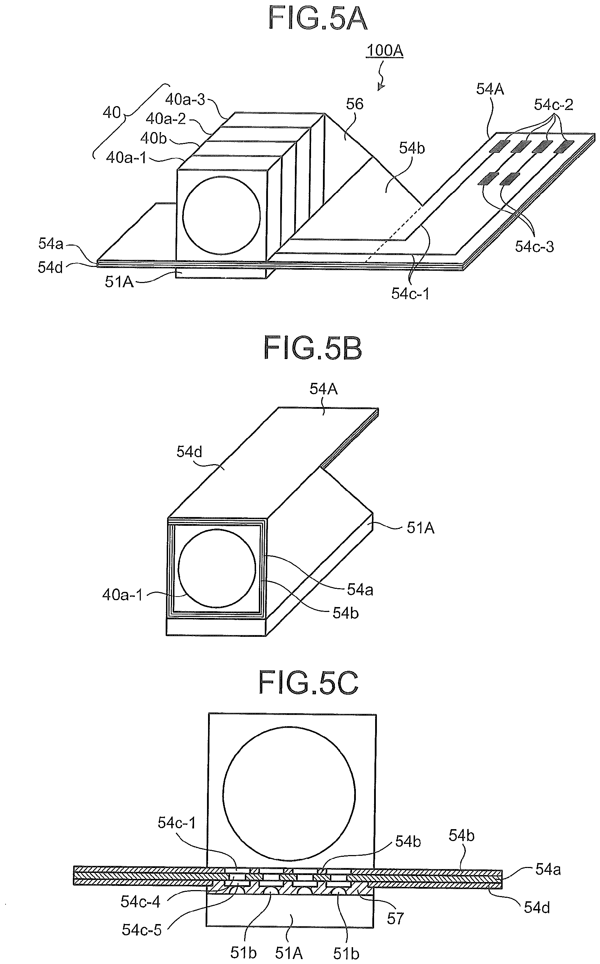

FIG. 5A is a perspective view for explaining arrangement of a moisture-proof member of an imaging apparatus according to a first modification of the first embodiment of the present invention (before arrangement), and FIG. 5B is a perspective view for explaining arrangement of the moisture-proof member of the imaging apparatus according to a first modification of the first embodiment of the present invention (after arrangement). FIG. 5C is an enlarged view of a connection portion of an imaging element and a moisture-proof member 54A according to the first modification of the first embodiment of the present invention. In FIG. 5A and FIG. 5B, illustrations of the electronic components and the like are omitted.

An imaging apparatus 100A includes the lens unit 40 that condenses incident light, a prism 56 that reflects the light condensed by the lens unit 40, and an imaging element 51A that generates an image signal based on the light incident from the prism 56.

The imaging element 51A is a transverse type, in which a principal surface on which the light receiving unit 51a is provided is arranged horizontally, that is, the principal surface is arranged parallel to the optical axis of the lens unit 40, and the prism 56 is arranged and bonded onto the light receiving unit 51a.

Further, electrode pads 51b are provided at a proximal end of the imaging element 51A, and connected to element electrodes 54c-5 of the moisture-proof member 54A to be described later.

The moisture-proof member 54A is a flexible connector including the base material 54a with moisture-proof property, the adhesive layer 54b provided on a surface of the base material 54a, a wiring layer 54c provided on the adhesive layer 54b, and a light shielding layer 54d provided on a surface of the base material 54a opposite to the surface where the adhesive layer 54b is provided.

The wiring layer 54c includes wiring patterns 54c-1 for wiring electrode portions to be described later, cable electrodes 54c-2 for connecting signal cables, component electrodes 54c-3 for connecting electronic components, through vias 54c-4, and the element electrodes 54c-5 for connecting the electrode pads 51b.

As illustrated in FIG. 5A, the moisture-proof member 54A is arranged between the imaging element 51A and the lens unit 40, bent at a dotted-line portion, and arranged so as to come in contact with a part of the side surfaces of the lens unit 40 and the prism 56 (see FIG. 5B). The moisture-proof member 54A is tightly bonded to the lens unit 40 and the prism 56 by the adhesive layer 54b, and prevents moisture from entering a gap between the optical element 40a-1 and the spacer 40b, a gap between the spacer 40b and the optical element 40a-2, a gap between the optical element 40a-2 and the optical element 40a-3, and a gap between the optical element 40a-3 and the prism 56.

The element electrodes 54c-5 of the moisture-proof member 54A are arranged on and electrically and mechanically connected to the electrode pads 51b of the imaging element 51A. Peripheries of the connection portions are sealed by sealing resin 57. By providing the wiring layer 54c, such as the element electrodes 54c-5, the through vias 54c-4, and the wiring patterns 54c-1, on the moisture-proof member 54A, the moisture-proof member 54A can be used as an FPC board.

Further, the moisture-proof member 54A includes the light shielding layer 54d, and therefore, it is possible to prevent stray light from entering the lens unit 40 and the prism 56 and reduce influence of external light.

### (Second Embodiment)

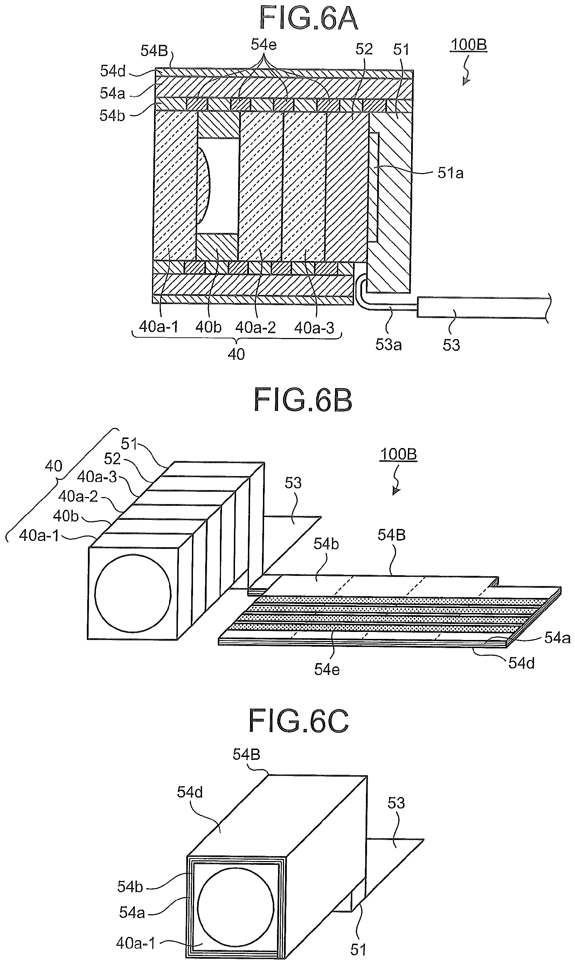

FIG. 6A is a cross-sectional view of an imaging apparatus according to a second embodiment of the present invention. FIG. 6B is a perspective view for explaining arrangement of a moisture-proof member of the imaging apparatus in the second embodiment of the present invention (before arrangement), and FIG. 6C is a perspective view for explaining arrangement of the moisture-proof member of the imaging apparatus in the second embodiment of the present invention (after arrangement).

In an imaging apparatus 100B of the second embodiment, a moisture-proof member 54B is a flexible connector including the base material 54a with moisture-proof property, moisture-resistant rings 54e that are provided on a surface of the base material 54a and seals connection boundaries of the lens unit 40, an adhesive layer 54b that is provided so as to cover the surface of the base material 54a and the moisture-resistant ring, and the light shielding layer 54d that is provided on a surface of the base material 54a opposite to the surface where the adhesive layer 54b is provided.

The moisture-resistant rings 54e are provided at certain positions so as to seal a connection boundary between the optical element 40a-1 and the spacer 40b, a connection boundary between the spacer 40b and the optical element 40a-2, a connection boundary between the optical element 40a-2 and the optical element 40a-3, a connection boundary between the optical element 40a-3 and the cover glass 52, and a connection boundary between the cover glass 52 and the imaging element 51. It is sufficient that the moisture-resistant rings 54e are made of metal, such as copper or cold, or resin with high moisture resistance, such as benzocyclobutene (BCB), and molded by a photolithography method, similarly to the wiring layer.

The moisture-proof member 54B is bent as indicated by dotted lines in FIG. 6B, and arranged so as to be in contact with a part of the side surfaces of the lens unit 40, the side surfaces of the cover glass 52, and the side surfaces of the imaging element 51 as illustrated in FIG. 6C. The moisture-proof member 54B is bent so as to come in contact with the side surfaces of the lens unit 40 and the like, and the moisture-resistant rings 54e seal the connection boundary between the optical element 40a-1 and the spacer 40b, the connection boundary between the spacer 40b and the optical element 40a-2, the connection boundary between the optical element 40a-2 and the optical element 40a-3, the connection boundary between the optical element 40a-3 and the cover glass 52, and the connection boundary between the cover glass 52 and the imaging element 51; therefore, it is possible to further improve the moisture-proof performance of the imaging apparatus 100B.

While the moisture-resistant member 54B and the FPC board 53 are configured as separate members in the second embodiment, they may be configured as an integrated flexible connector. When the moisture-resistant member and the FPC board are integrated, it is sufficient that the moisture-resistant rings 54e are formed at the same time of performing wiring on the FPC board.

### (Third Embodiment)

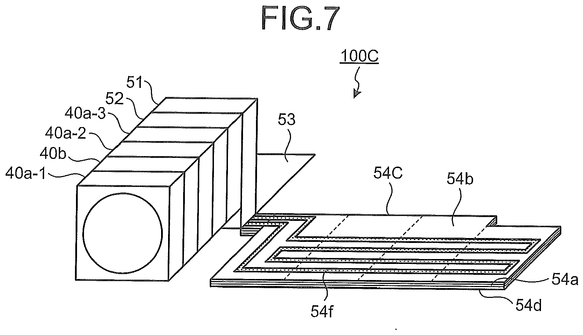

FIG. 7 is a perspective view for explaining arrangement of a moisture-proof member of the imaging apparatus in a third embodiment of the present invention (before arrangement).

In an imaging apparatus 100C of the third embodiment, a moisture-proof member 54C is a flexible connector including the base material 54a with moisture-proof property, a heater wiring 54f that is provided on a surface of the base material 54a and that heats the lens unit 40, the adhesive layer 54b that is provided so as to cover the surface of the base material 54a and the heater wiring 54f, and the light shielding layer 54d that is provided on a surface of the base material 54a opposite to the surface where the adhesive layer 54b is provided.

The heater wiring 54f is provided on all over the adhesive layer 54b so as to heat all peripheries of the side surfaces of the lens unit 40. It is sufficient that the heater wiring 54f is formed by a photolithography method, similarly to the wiring layer.

The moisture-proof member 54C is bent as indicated by dotted lines in FIG. 7, and arranged so as to come in contact with a part of the side surfaces of the lens unit 40, the side surfaces of the cover glass 52, and the side surfaces of the imaging element 51. The moisture-proof member 54C is bent so as to come in contact with the side surfaces of the lens unit 40 and the like, and power is supplied to the heater wiring 54f to heat the lens unit 40; therefore, it is possible to further improve the moisture-proof performance of the imaging apparatus 100C.

While the moisture-resistant member 54C and the FPC board 53 are configured as separate members in the third embodiment, they may be configured as an integrated flexible connector. When the moisture-resistant member and the FPC board are integrated, it is sufficient that the heater wiring 54f is formed at the same time of performing wiring on the FPC board.

### Reference Signs List

1 ENDOSCOPIC SYSTEM
2 ENDOSCOPE
3 INSERTION PORTION
3A DISTAL END PORTION
3B BENDABLE PORTION
3C FLEXIBLE TUBE
4 OPERATING UNIT
4a TREATMENT TOOL INSERTION OPENING
4b BENDING OPERATION KNOB
6 UNIVERSAL CORD
7 CONNECTOR
8 PROCESSOR
9 LIGHT SOURCE DEVICE
10 DISPLAY DEVICE
34 BENDING TUBE
35 DISTAL END FIXING PORTION
36 TREATMENT TOOL CHANNEL
40 LENS UNIT
40a-1, 40a-2, 40a-3 OPTICAL ELEMENT
40b SPACER
50 IMAGING UNIT
51, 51A IMAGING ELEMENT
51a LIGHT RECEIVING UNIT
51b ELECTRODE PAD
52 COVER GLASS
53 FLEXIBLE PRINTED CIRCUIT BOARD
54, 54A, 54B, 54C MOISTURE-PROOF MEMBER
54a BASE MATERIAL
54b ADHESIVE LAYER
54c WIRING LAYER
54c-1 WIRING PATTERN
54c-2 CABLE ELECTRODE
54c-3 COMPONENT ELECTRODE
54c-4 THROUGH VIA
54c-5 ELEMENT ELECTRODE
54d LIGHT SHIELDING LAYER
54e MOISTURE-RESISTANT RING
54f HEATER WIRING
55 ELECTRONIC COMPONENT
56 PRISM
57 SEALING RESIN
60 SIGNAL CABLE
100, 100A, 100B, 100C IMAGING APPARATUS

## Claims

1. An imaging apparatus comprising:
a lens unit in which a plurality of optical elements including one or more wafer-level lenses and a spacer are laminated;
an imaging element configured to receive light incident from the lens unit, perform photoelectric conversion on the light, and generate an image signal; and
a moisture-proof member that is bent and arranged so as to come in contact with at least a part of side surfaces of the lens unit to prevent moisture from entering a gap between the optical element and the spacer and/or a gap between the optical elements.

2. The imaging apparatus according to claim 1, wherein the moisture-proof member is a flexible printed circuit board and bonded to the side surfaces of the lens unit by an adhesive agent.

3. The imaging apparatus according to claim 1, wherein the moisture-proof member is a flexible connector including a base material with moisture-proof property and an adhesive layer provided on a surface of the base material.

4. The imaging apparatus according to claim 1, wherein the moisture-proof member is a flexible connector including a base material with moisture-proof property, and an adhesive layer and a wiring layer that are provided on a surface of the base material.

5. The imaging apparatus according to any one of claims 1 to 3, wherein the imaging element is arranged such that a principal surface, on which the light receiving unit is provided, is arranged perpendicular to an optical axis of the lens unit.

6. The imaging apparatus according to claim 4, wherein the moisture-proof member is a flexible connector including a base material with moisture-proof property, an adhesive layer provided on a surface of the base material, and a moisture-resistant ring for sealing a boundary between the optical element and the spacer and/or a boundary between the optical elements.

7. The imaging apparatus according to claim 4, wherein the moisture-proof member is a flexible connector including a base material with moisture-proof property, an adhesive layer provided on a surface of the base material, and a heater wiring that heats the lens unit.

8. The imaging apparatus according to claim 1, further comprising:
a prism provided on the light receiving unit and configured to reflect light incident from the lens unit toward the light receiving unit, wherein
the imaging element is arranged such that a principal surface on which the light receiving unit is provided is arranged parallel to an optical axis of the lens unit, and the lens unit is arranged on the imaging element,
the moisture-proof member is a flexible connector that includes a base material with moisture-proof property, an adhesive layer provided on a surface of the base material, and a wiring layer, and is bent and arranged such that the adhesive layer and the wiring layer come in contact with the lens unit, and
the wiring layer is connected to an electrode pad of the imaging element via a through via provided in the moisture-proof member.

9. The imaging apparatus according to any one of claims 1 to 8, wherein the moisture-proof member includes a light shielding layer on a surface opposite to a surface that comes in contact with the lens unit.

10. The imaging apparatus according to any one of claims 1 to 7 and 9, further comprising:
a flexible printed circuit board on which a signal cable and an electronic component are mounted and which is connected to an electrode pad of the imaging element, wherein
the moisture-proof member is integrated with the flexible printed circuit board.

11. An endoscope comprising:
the imaging apparatus according to any one of claims 1 to 10; and
an insertion portion that includes a tubular distal end portion made of a rigid material and that is insertable into a subject, wherein
the insertion portion is provided with the imaging apparatus in an internal space of the distal end portion.
